(19) Europäisches Patentamt
European Patent Office
Office européen des brevets

(11) Veröffentlichungsnummer: **0 075 752**
**B1**

(12) # EUROPÄISCHE PATENTSCHRIFT

(45) Veröffentlichungstag der Patentschrift: **12.03.86**

(21) Anmeldenummer: **82108196.5**

(22) Anmeldetag: **06.09.82**

(51) Int. Cl.[4]: **C 07 D 213/64,** C 07 D 213/65, C 07 D 213/68, C 07 D 213/70, C 07 D 213/74, A 61 K 31/44, A 61 K 31/535

(54) **Substituierte 2-Phenyl-2-(pyridyloxy)-ethylamine und isostere Verbindungen, Verfahren zu ihrer Herstellung und Verwendung.**

(30) Priorität: **28.09.81 DE 3138550**

(43) Veröffentlichungstag der Anmeldung: **06.04.83 Patentblatt 83/14**

(45) Bekanntmachung des Hinweises auf die Patenterteilung: **12.03.86 Patentblatt 86/11**

(84) Benannte Vertragsstaaten: **AT BE CH DE FR IT LI LU NL SE**

(56) Entgegenhaltungen:
**DE - A - 2 252 882**
**US - A - 4 011 324**

**Chemical Abstracts Band 90, Nr. 25, 18. Juni 1979, Columbus, Ohio, USA; Seite 606, Spalten 1, 2, Zusammenfassung Nr. 203875v**

(73) Patentinhaber: **BOEHRINGER INGELHEIM KG, D-6507 Ingelheim am Rhein (DE)**

(72) Erfinder: **Schneider, Claus, Dr., Albrecht-Dürer-Strasse 19, D-6507 Ingelheim/Rhein (DE)**
Erfinder: **Walther, Gerhard, Dr., Pfarrer-Heberer-Strasse 37, D-6530 Bingen/Rhein (DE)**
Erfinder: **Weber, Karl-Heinz, Dr., Kaiser-Karl-Strasse 11, D-6535 Gau-Algesheim (DE)**
Erfinder: **Bechtel, Wolf Dietrich, Dr., Mühlstrasse 3, D-6531 Appenheim (DE)**
Erfinder: **Böke-Kuhn, Karin, Dr., Beethovenstrasse 11, D-6535 Gau-Algesheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 075 752 B1

## Beschreibung

Die Erfindung betrifft neue substituierte 2-Phenyl -2- (pyridyloxy) -ethylamine und isostere Verbindungen der allgemeinen Formel

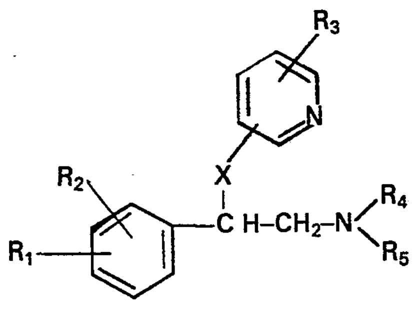

(I)

worin

$R_1$ und $R_2$, die gleich oder verschieden sein können, Wasserstoff, ein Halogenatom, Methyl, Methoxy, Amino oder Nitro,

$R_3$ Wasserstoff, Halogen oder Methyl und

$R_4$ und $R_5$, die gleich oder verschieden sein können, Wasserstoff oder eine Alkylgruppe mit 1–2 Kohlenstoffatomen bedeuten oder zusammen mit dem Stickstoffatom einen Pyrrolidino- oder Morpholinoring bilden können und

X O, NH oder S bedeutet, ihre Säureadditionssalze, Verfahren zu ihrer Herstellung und ihre Verwendung als Wirkstoffe in Arzneimitteln.

Die neuen Verbindungen können erhalten werden durch Umsetzung eines Phenylethylamins der allgemeinen Formel

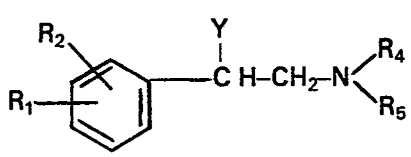

(II)

worin $R_1$, $R_2$, $R_4$ und $R_5$ die oben angegebene Bedeutung haben und Y ein Halogenatom oder die Hydroxygruppe bedeutet beziehungsweise eines Säureadditionssalzes dieser Verbindung mit einem Pyridinderivat der allgemeinen Formel

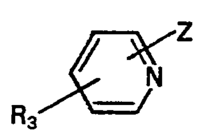

(III)

worin $R_3$ die oben angegebene Bedeutung hat und Z die Hydroxy-, Amino- oder Mercaptogruppe bedeutet.

Bei Ausgangsstoffen der allgemeinen Formel II, in denen Y die Hydroxygruppe bedeutet, erfolgt die Kondensation mit einer Verbindung der allgemeinen Formel III im sauren Medium bei erhöhten Temperaturen. Für diese Umsetzung kommen starke anorganische oder organische Säuren, z.B. konzentrierte Mineralsäuren wie Chlorwasserstoffsäure, Bromwasserstoffsäure, Schwefelsäure, Polyphosphorsäure, ferner Essigsäureanhydrid, Phosphorpentoxyd oder Trifluoressigsäure in Frage.

Phenylethylamine der allgemeinen Formel II, in denen Y ein Halogenatom bedeutet, werden vorzugsweise unter Verwendung eines basischen Stoffes und vorzugsweise unter Phasen-Transfer-Bedingungen (d.h. unter Zusatz eines oder mehrerer langkettigen tertiären Amine wie einem Benzyl-tributyl-ammonium-halogenid oder einem Tetrabutyl-ammonium-halogenid oder unter Zusatz von Benzyl-triphenyl-phosphoniumchlorid) mit dem Pyridinderivat der allgemeinen Formel III umgesetzt.

Als basische Stoffe kommen in Frage anorganische Basen wie Alkali- oder Erdalkalihydroxyde oder -carbonate, organische Basen wie Pyridin, Piperidin, tertiäre Amine oder auch Alkalialkoholate.

Verbindungen der allgemeinen Formel I, in denen $R_4$ und/oder $R_5$ Wasserstoff bedeuten, lassen sich in an sich bekannter Weise, z.B. durch Umsetzung mit einem Dialkylsulfat oder einem Alkylhalogenid, alkylieren.

Nach dem oben beschriebenen Verfahren können beispielsweise die folgenden Endprodukte, gegebenenfalls in Form ihrer Säureadditionssalze, erhalten werden:

2-Phenyl-2-(pyridyl-(3)-oxy) -N,N-dimethyl-ethylamin,

2-Phenyl-2-(pyridyl-(2)-oxy) -N,N-dimethyl-ethylamin,

2-(p-Bromphenyl)-2-(pyridyl-(2)-oxy) -N,N-dimethylethylamin,

2-(p-Bromphenyl)-2-(pyridyl)-3)-oxy) -N,N-dimethyl-ethylamin,

2-(p-Bromphenyl) -2- (2-chlor-pyridyl-(3)-oxy) -N,N- dimethyl-ethylamin,

2-(p-Bromphenyl) -2- (5- chlor-pyridyl-(2)-oxy) -N,N- dimethyl-ethylamin,

2-(p-Bromphenyl)-2- (6-chlor-pyridyl-(2)-oxy) -N,N- dimethyl-ethylamin,

2-(p-Bromphenyl)-2- (pyridyl-(4)-oxy) -N,N-dimethyl-ethylamin,

2-(p-Chlorphenyl)-2- (pyridyl-(3)-oxy)-N,N-dimethyl-ethylamin,

2-(m,p-Dichlorphenyl) -2- (pyridyl-(3)-oxy)-N,N-dimethyl-ethylamin,

2-Phenyl -2- (pyridyl-(2)- amino) -N,N-dimethyl-ethylamin,

2-Phenyl -2- (pyridyl-(3)-amino) -N,N-dimethyl-ethylamin,

2-(p-Bromphenyl) -2- (pyridyl-(2)-amino)-N-methyl-ethylamin,

2-(p-Bromphenyl) -2- (pyridyl-(2)-amino)-N,N-dimethyl-ethylamin,

2-(p-Bromphenyl) -2- (pyridyl-(3)-amino)-N,N-dimethyl-ethylamin,

2-Phenyl -2- (4-methyl-pyridyl-(2)-amino)-N,N-dimethyl-ethylamin,

2-(p-Bromphenyl) -2- (5-methyl-pyridyl-(2)-amino) -N,N-dimethyl-ethylamin,

2-(p-Bromphenyl) -2- (6-methyl-pyridyl-(2)-amino) -N,N-dimethyl-ethylamin,

2-(4-p-Bromphenyl) -2- (5-chlor-pyridyl-(2)-amino) -N,N-dimethyl-ethylamin,

2-(4-p-Bromphenyl)- 2-(pyridyl-(2)-thio)-N,N-dimethyl-ethylamin,

2-(p-Bromphenyl) -2- (pyridyl-(3)-oxy) morpholino-ethylamin,
2-(p-Bromphenyl) -2- (pyridyl-(3)-oxy) -pyrrolidino-ethylamin,
2-(p-Bromphenyl) -2- (pyridyl-(2)-amino)-morpholino-ethylamin,
2-(p-Bromphenyl) -2- (pyridyl-(2)-amino)-pyrrolidino-ethylamin,
2-(p-Methoxyphenyl) -2- (pyridyl-(3)-oxy)-N,N-dimethyl-ethylamin,
2-(p-Hydroxyphenyl) -2- (pyridyl-(3)-oxy)-N,N-dimethyl-ethylamin,
2-(p-Tolyl) -2- (pyridyl-(3)-oxy)- N,N- diethyl-ethylamin,
2-(p-Nitrophenyl) -2- (pyridyl-(2)-oxy)-N,N-dimethylethylamin,
2-(4-Aminophenyl) -2- (pyridyl-(2)-oxy)-N,N-dimethyl-ethylamin,
2-(m,p-Dichlorphenyl) -2- (pyridyl-(3)-amino) -N,N-dimethyl-ethylamin,
2-(m,p-Dimethoxyphenyl) -2- (pyridyl-(3)-oxy) -N,N-dimethyl-ethylamin.

Die Ausgangsstoffe sind gängige Chemikalien, die käuflich erworben werden oder aber nach allgemein bekannten Verfahren hergestellt werden können.

Die Endprodukte der allgemeinen Formel I können gewünschtenfalls nach üblichen Methoden in ihre physiologisch unbedenklichen Säureadditionssalze überführt werden.

Als Säuren eignen sich hierfür sowohl anorganische Säuren wie Halogenwasserstoffsäuren, Schwefelsäure, Phosphorsäure und Aminosulfonsäure, als auch organische Säuren wie Ameisensäure, Essigsäure, Propionsäure, Milchsäure, Glykolsäure, Glukonsäure, Maleinsäure, Bernsteinsäure, Weinsäure, Benzoesäure, Salicylsäure, Zitronensäure, Ascorbinsäure, p-Toluolsulfonsäure oder Oxyäthansulfonsäure.

Die neuen Stoffe der allgemeinen Formel I und ihre Säureadditionssalze stellen wertvolle Pharmazeutika dar; sie sind in den für Antidepressiva spezifischen biochemischen und pharmakologischen Testanordnungen gut wirksam. So besitzen sie die Fähigkeit, die an der Maus durch Tetrabenzain induzierte Ptosis zu hemmen; die $ED_{50}$ liegt in der Grössenordnung von 1 mg/kg. Dieser Test dient als Standardtest auf antidepressive Eigenschaften (International Journal of Neuropharmacology 8, 73 (1969)).

Auch im Test auf Reserpinantagonismus, einer Aufhebung des durch Reserpin verursachten hypothermischen Effektes durch eine antidepressiv wirksame Substanz, haben die erfindungsgemässen Verbindungen ausserordentlich günstige Wirkung gezeigt. Ausserdem wurde festgestellt, dass sie die Wiederaufnahme von Serotonin und Adrenalin in die Neuronen hemmen.

Die neuen Verbindungen sind besonders wertvoll durch eine von den bisher bekannten Antidepressiva abweichende Struktur; bekannten Handelsprodukten sind sie in ihrer Wirkung bei geringerer Toxizität gleichwertig oder überlegen.

Insbesondere seien solche Verbindungen der allgemeinen Formel I hervorgehoben, in denen $R_1$ ein Bromatom in p-Stellung, X ein Sauerstoffatom oder die Aminogruppe, $R_2$ Wasserstoff und $R_3$ und $R_4$ Methylgruppen darstellen. Besonders hervorgehoben seien die Verbindungen
2-(p-Bromphenyl) -2- (pyridyl-(3)-oxy)-N,N-dimethylethylamin,
2-(p-Bromphenyl) -2- (pyridyl-(2)- amino)-N,N-dimethylethylamin,
2-(p-Bromphenyl) -2- (pyridyl-(3)-amino)-N,N-dimethyl-ethylamin.

Die folgenden Beispiele dienen zur näheren Erläuterung der Erfindung:

Beispiel 1

2-p-Bromphenyl-2- (3-pyridyloxy) -N,N- dimethyl-ethylamin-dihydrochlorid

29,9 g 2-Chlor-2-p-bromphenyl- N,N-dimethyl-ethylamin-hydrochlorid (0,1 Mol) und 14,3 g 3-Hydroxypyridin (0,15 Mol) werden in 150 ml 25%iger Natronlauge und 150 ml Toluol mit 0,5 g Benzyltriphenylphosphoniumchlorid 16 Stunden unter Rückfluss zum Sieden erhitzt. Nach dem Abdestillieren des Lösungsmittels von der organischen Phase verbleibt ein Rückstand, der in Alkohol gelöst wird. Mit Salzsäure entsteht das Dihydrochlorid der Titelverbindung. Zur Reinigung wird die Titelverbindung in Cyclohexan mit Aktivkohle und Kieselgur behandelt. Die Ausbeute an Dihydrochlorid beträgt 20 g (44% d.Th.). Fp. 128–129 °C (Ethanol).

Beispiel 2

2-p-Bromphenyl-2- (2-pyridylamino) -N,N-dimethyl-ethylamin-dioxalat

24,5 g 2-p-Bromphenyl-N,N- dimethylethanolamin (0,1 Mol) und 11 g 2-Aminopyridin (0,11 Mol) werden in 50 ml Methansulfonsäure 30 Minuten auf 60 °C erwärmt. Die noch warme Reaktionsmischung wird auf Eis gegeben, mit Ammoniak alkalisch gemacht und mit Essigester extrahiert. Die Reinigung erfolgt säulenchromatographisch über Kieselgel/Methylenchlorid-Essigester-Methanol. Durch Zugabe von alkoholischer Oxalsäure entsteht das kristalline Dioxalat der Titelverbindung. Die Ausbeute beträgt 21 g (42% d.Th.). Fp. 170–171 °C.

Nach dem vorstehend beschriebenen Verfahren wurden folgende Verbindungen der allgemeinen Formel I erhalten:

| Beispiel Nr.: | $R_1$ | $R_2$ | $R_3$ | $R_4$ | $R_5$ | X | Verknüpfung Pyridyl | Fp. °C |
|---|---|---|---|---|---|---|---|---|
| 3 | 4-Br | H | H | H | $CH_3$ | NH | 2 | 181–182 |
| 4 | H | H | H | $CH_3$ | $CH_3$ | O | 2 | 161–162 |
| 5 | H | H | H | $CH_3$ | $CH_3$ | O | 3 | 151–152 |
| 6 | H | H | H | $CH_3$ | $CH_3$ | NH | 2 | 129–130 |
| 7 | H | H | H | $CH_3$ | $CH_3$ | NH | 3 | 123–125 |
| 8 | 4-Br | H | 2-Cl | $CH_3$ | $CH_3$ | O | 3 | 135–136 |
| 9 | 4-Br | H | 5-Cl | $CH_3$ | $CH_3$ | O | 2 | 234–235 |
| 10 | 4-Br | H | 6-Cl | $CH_3$ | $CH_3$ | O | 2 | 193–194 |
| 11 | H | H | 4-$CH_3$ | $CH_3$ | $CH_3$ | NH | 2 | 139–140 |
| 12 | 4-Br | H | 5-Cl | $CH_3$ | $CH_3$ | NH | 2 | 134–136 |
| 13 | 4-Br | H | 5-$CH_3$ | $CH_3$ | $CH_3$ | NH | 2 | 103–104 |
| 14 | 4-Br | H | 6-$CH_3$ | $CH_3$ | $CH_3$ | NH | 2 | 135–136 |
| 15 | 4-Br | H | H | $CH_3$ | $CH_3$ | NH | 3 | 144–145 |
| 16 | 4-Br | H | H | $CH_3$ | $CH_3$ | O | 2 | 162–163 |
| 17 | 4-Br | H | H | $CH_3$ | $CH_3$ | S | 2 | 190–191 |
| 18 | 4-Br | H | H | Morpholin | | O | 3 | 95– 97 |
| 19 | 4-Br | H | H | Morpholin | | NH | 2 | 194–195 |
| 20 | 4-Br | H | H | Pyrrolidin | | O | 3 | 152–153 |
| 21 | 4-Br | H | H | Pyrrolidin | | NH | 2 | 117–119 |
| 22 | 4-Br | H | H | $CH_3$ | $CH_3$ | O | 4 | 112 (Zers.) |
| 23 | 4-Cl | H | H | $CH_3$ | $CH_3$ | O | 3 | 126–129(Zers.) |
| 24 | 3-Cl | 4-Cl | H | $CH_3$ | $CH_3$ | O | 3 | 175–176 |
| 25 | 4-$OCH_3$ | H | H | $CH_3$ | $CH_3$ | O | 3 | 103–105 |
| 26 | 3-Cl | 4-Cl | H | $CH_3$ | $CH_3$ | NH | 3 | 152–153 |
| 27 | 3-$OCH_3$ | H | H | H | H | O | 3 | 222–223 |
| 28 | 4-$CH_3$ | H | H | $CH_3$ | $CH_3$ | O | 3 | 180–181 |
| 29 | 4-Br | H | 4-$CH_3$ | $CH_3$ | $CH_3$ | NH | 2 | 219–220 |

Formulierungsbeispiele

a) Dragees

1 Drageekern enthält:

| | |
|---|---|
| Wirkstoff gemäss der Erfindung | 25,0 mg |
| Milchzucker | 50,0 mg |
| Maisstärke | 22,0 mg |
| Gelatine | 2,0 mg |
| Magnesiumstearat | 1,0 mg |
| | 100,0 mg |

Herstellung:

Die Mischung der Wirksubstanz mit Milchzukker und Maisstärke wird mit einer 10%igen wässrigen Gelatinelösung durch ein Sieb mit 1 mm Maschenweite granuliert, bei 40 °C getrocknet und nochmals durch ein Sieb getrieben. Das so erhaltene Granulat wird mit Magnesiumstearat gemischt und verpresst. Die so erhaltenen Kerne werden in üblicher Weise mit einer Hülle überzogen, die mit Hilfe einer wässrigen Suspension von Zucker, Titandioxyd, Talkum und Gummi arabicum aufgebracht wird. Die fertigen Dragees werden mit Bienenwachs poliert. Dragee-Endgewicht: 200 mg

b) Tabletten

| | |
|---|---|
| Wirkstoff gemäss der Erfindung | 10,0 mg |
| Milchzucker | 40,0 mg |
| Maisstärke | 44,0 mg |
| lösliche Stärke | 5,0 mg |
| Magnesiumstearat | 1,0 mg |
| | 100,0 mg |

Herstellung:

Wirkstoff und Magnesiumstearat werden mit einer wässrigen Lösung der löslichen Stärke granuliert, das Granulat getrocknet und innig mit Milchzucker und Maisstärke vermischt. Das Gemisch wird sodann zu Tabletten von 100 mg Gewicht verpresst, die je 10 mg Wirkstoff enthalten.

c) Suppositorien

1 Zäpfchen enthält:

| | |
|---|---|
| Wirkstoff gemäss der Erfindung | 10,0 mg |
| Zäpfchenmasse | 1.690,0 mg |

Herstellung:

Die feingepulverte Substanz wird mit Hilfe eines Eintauch-Homogenisators in die geschmolzene und auf 40 °C abgekühlte Zäpfchenmasse eingerührt. Die Masse wird bei 35 °C in leicht vorgekühlte Formen gegossen.

d) Ampullen (Injektionslösungen)

Zusammensetzung:

| | |
|---|---|
| Wirkstoff gemäss der Erfindung | 5,0 Gew.-Teile |
| Natriumpyrosulfit | 1,0 Gew.-Teile |
| Dinatriumsalz der Ethylendiamintetraessigsäure | 0,5 Gew.-Teile |
| Natriumchlorid | 8,5 Gew.-Teile |
| doppelt destilliertes Wasser ad | 1000,0 Gew.-Teile |

Herstellung:

Der Wirkstoff und die Hilfsstoffe werden in einer ausreichenden Menge Wasser gelöst und mit

der notwendigen Menge Wasser auf die gewünschte Konzentration gebracht. Die Lösung wird filtriert und unter aseptischen Bedingungen in 1 ml Ampullen abgefüllt. Zuletzt werden die Ampullen sterilisiert und verschlossen. Jede Ampulle enthält 5,0 mg Wirkstoff.

**Patentansprüche**

1. Substituierte 2-Phenyl-2- (pyridyloxy)-ethylamine und isostere Verbindungen der allgemeinen Formel

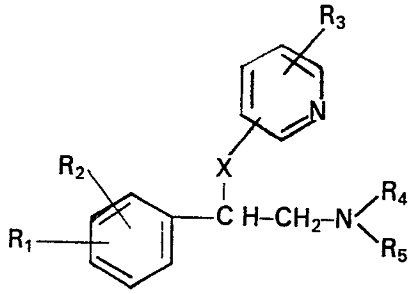

(I)

worin

$R_1$ und $R_2$, die gleich oder verschieden sein können, Wasserstoff, ein Halogenatom, Methyl, Methoxy, Amino oder Nitro,

$R_3$ Wasserstoff, Halogen oder Methyl und

$R_4$ und $R_5$, die gleich oder verschieden sein können, Wasserstoff oder eine Alkylgruppe mit 1–2 Kohlenstoffatomen bedeuten oder zusammen mit dem Stickstoffatom einen Pyrrolidino- oder Morpholinoring bilden können und

X O, NH oder S bedeutet sowie deren physiologisch verträgliche Säureadditionssalze.

2. 2-(p-Bromphenyl) -2- (pyridyl-(3)-oxy)-N,N- dimethylethylamin und dessen physiologisch verträgliche Säureadditionssalze.

3. 2-(p-Bromphenyl) -2- (pyridyl-(2)-amino)-N,N-dimethyl-ethylamin und dessen physiologisch verträgliche Säureadditionssalze.

4. 2-(p-Bromphenyl) -2- (pyridyl-(3)-amino)-N,N-dimethyl-ethylamin und dessen physiologisch verträgliche Säureadditionssalze.

5. Verfahren zur Herstellung neuer substituierter 2-Phenyl-2- (pyridyloxy) -ethylamine und deren isosteren Verbindungen der allgemeinen Formel

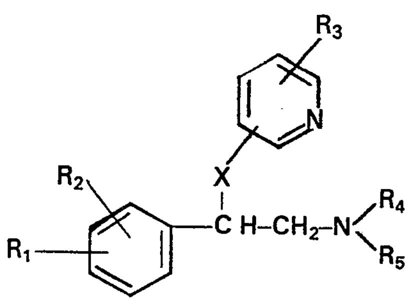

(I)

worin

$R_1$ und $R_2$, die gleich oder verschieden sein können, Wasserstoff, ein Halogenatom, Methyl, Methoxy, Amino oder Nitro,

$R_3$ Wasserstoff, Halogen oder Methyl und

$R_4$ und $R_5$, die gleich oder verschieden sein können, Wasserstoff oder eine Alkylgruppe mit 1–2 Kohlenstoffatomen bedeuten oder zusammen mit dem Stickstoffatom einen Pyrrolidino- oder Morpholinoring bilden können und

X O, NH oder S bedeutet und deren physiologisch verträgliche Säureadditionssalze, dadurch gekennzeichnet, dass man ein Phenylethylamin der allgemeinen Formel

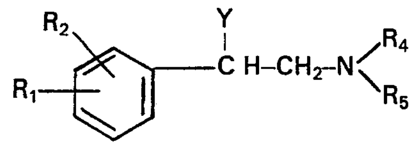

(II)

worin

$R_1$, $R_2$, $R_4$ und $R_5$ die oben angegebene Bedeutung haben und Y ein Halogenatom oder die Hydroxygruppe bedeutet, beziehungsweise ein Säureadditionssalz dieser Verbindung mit einem Pyridinderivat der allgemeinen Formel

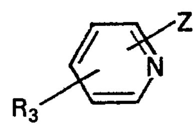

(III)

worin

$R_3$ Wasserstoff, ein Halogenatom oder die Methylgruppe und

Z die Hydroxy-, Amino- oder Mercaptogruppe bedeutet, umsetzt und das so erhaltene Endprodukt gegebenenfalls in ein physiologisch verträgliches Säureadditionssalz überführt.

6. Verfahren nach Anspruch 5, dadurch gekennzeichnet, dass man eine Verbindung der allgemeinen Formel I, worin $R_4$ und/oder $R_5$ Wasserstoff bedeuten, alkyliert.

7. Pharmazeutische Zusammensetzungen, enthaltend als Wirkstoffe Verbindungen der allgemeinen Formel I in Kombination mit üblichen Hilfs- und/oder Trägerstoffen.

8. Pharmazeutische Zusammensetzungen gemäss Anspruch 7 zur Behandlung depressiver Zustände verschiedener Genese.

**Revendications**

1. 2-phényl-2- (pyridyloxy) -éthylamines substituées et composés isostériques de formule générale

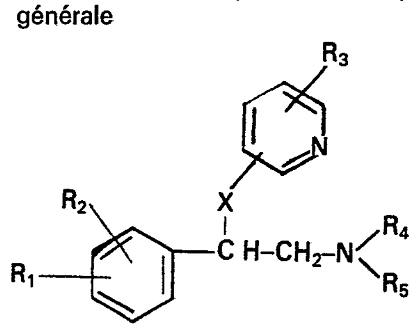

(I)

dans laquelle

$R_1$ et $R_2$, qui peuvent être identiques ou différents, représentent hydrogène, un atome d'halogène, méthyle, méthoxy, amino ou nitro,

$R_3$ représente hydrogène, halogène ou méthyle et

$R_4$ et $R_5$, qui peuvent être identiques ou différents, représentent hydrogène ou un groupe alcoyle avec 1–2 atomes de carbone ou peuvent former ensemble avec l'atome d'azote un cycle pyrrolidino ou morpholino et

X représente O, NH ou S, ainsi que leurs sels d'addition d'acides physiologiquement supportables.

2. La 2-(p-bromophényl) -2- (pyridyl-(3)-oxy) -N,N-diméthyl-éthylamine et ses sels d'addition d'acides physiologiquement supportables.

3. La 2-(p-bromophényl) -2- (pyridyl-(2)-amino) -N,N-diméthyl-éthylamine et ses sels d'addition d'acides physiologiquement supportables.

4. La 2-(p-bromophényl) -2- (pyridyl-(3)-amino) -N,N-diméthyl-éthylamine et ses sels d'addition d'acides physiologiquement supportables.

5. Procédé pour la préparation de nouvelles 2-phényl-2- (pyridyloxy) -éthylamines substituées et de leurs composés isostériques de formule générale

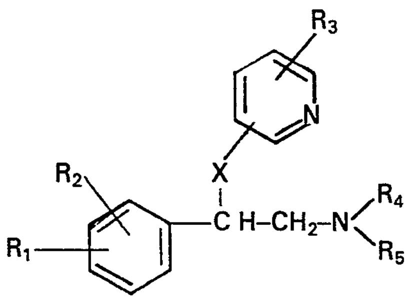

(I)

dans laquelle

$R_1$ et $R_2$, qui peuvent être identiques ou différents, représentent hydrogène, un atome d'halogène, méthyle, méthoxy, amino ou nitro,

$R_3$ représente hydrogène, halogène ou méthyle et

$R_4$ et $R_5$, qui peuvent être identiques ou différents, représentent hydrogène ou un groupe alcoyle avec 1–2 atomes de carbone ou peuvent former ensemble avec l'atome d'azote un cycle pyrrolidino ou morpholino et

X représente O, NH ou S, et de leurs sels d'addition d'acides physiologiquement supportables, caractérisé en ce que l'on fait réagir une phényléthylamine de formule générale

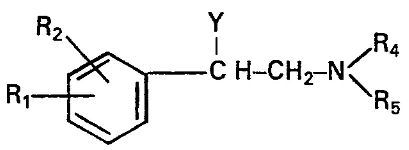

(II)

dans laquelle

$R_1$, $R_2$, $R_4$ et $R_5$ ont la signification indiquée plus haut et Y représente un atome d'halogène ou le groupe hydroxy, ou bien un sel d'addition d'acide de ce composé avec un dérivé de pyridine de formule générale

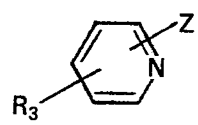

(III)

dans laquelle

$R_3$ représente hydrogène, un atome d'halogène ou le groupe méthyle et

Z représente le groupe hydroxy, amino ou mercapto et en ce que l'on transforme éventuellement le produit final ainsi obtenu en un sel d'addition d'acide physiologiquement supportable.

6. Procédé selon la revendication 5, caractérisé en ce que l'on alcoyle un composé de formule générale I dans laquelle $R_4$ et/ou $R_5$ représentent l'hydrogène.

7. Compositions pharmaceutiques contenant en tant que substances actives des composés de formule générale I en combinaison avec des adjuvants et/ou excipients usuels.

8. Compositions pharmaceutiques selon la revendication 7 pour le traitement des états dépressifs de différentes origines.

## Claims

1. Substituted 2-phenyl-2-(pyridyloxy) -ethylamine and isosteric compounds of general formula

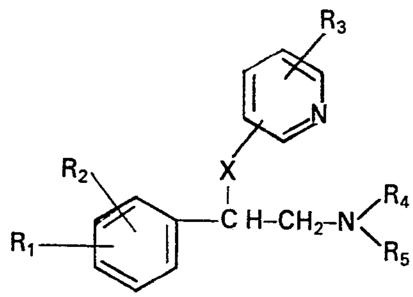

(I)

wherein

$R_1$ and $R_2$, which may be identical or different, represent hydrogen, a halogen atom, methyl, methoxy, amino or nitro,

$R_3$ represents hydrogen, halogen or methyl and

$R_4$ and $R_5$, which may be identical or different, represent hydrogen or an alkyl group having 1 to 2 carbon atoms or, together with the nitrogen atom, they may form a pyrrolidino or morpholino ring and

X represents O, NH or S, and the physiologically acceptable acid addition salts thereof.

2. 2-(p-Bromphenyl) -2- (pyridyl-(3)-oxy)-N,N-dimethylethylamine and the physiologically acceptable acid addition salts thereof.

3. 2-(p-Bromophenyl) -2- (pyridyl-(2)-amino) -N,N-dimethyl-ethylamine and the physiologically acceptable acid addition salts thereof.

4. 2-(p-Bromophenyl) -2- (pyridyl-(3)-amino) -N,N-dimethyl-ethylamine and the physiologically acceptable acid addition salts thereof.

5. Process for preparing new substituted

2-phenyl-2- (pyridyloxy) -ethylamine and the isosteric compounds of general formula

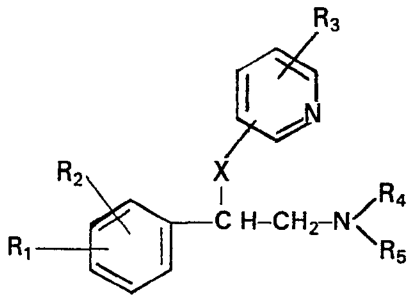

(I)

wherein

$R_1$ and $R_2$, which may be identical or different, represent hydrogen, a halogen atom, methyl, methoxy, amino or nitro,

$R_3$ represents hydrogen, halogen or methyl and

$R_4$ and $R_5$, which may be identical or different, represent hydrogen or an alkyl group having 1 to 2 carbon atoms or, together with the nitrogen atom, they may form a pyrrolidino or morpholino ring and

X represents O, NH or S, and the physiologically acceptable acid addition salts thereof, characterised in that a phenylethylamine of general formula

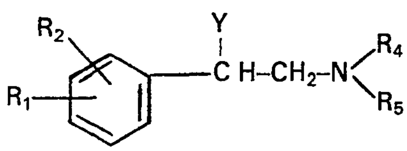

(II)

wherein

$R_1$, $R_2$, $R_4$ and $R_5$ are as hereinbefore defined and Y represents a halogen atom or a hydroxy group, or an acid addition salt of this compound is reacted with a pyridine derivative of general formula

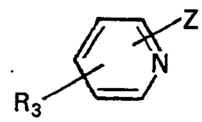

(III)

$R_3$ represents hydrogen, a halogen atom or a methyl group and

Z represents a hydroxy, amino or mercapto group, and the end product thus obtained is optionally converted into a physiologically acceptable acid addition salt thereof.

6. Process as claimed in claim 5, characterised in that a compound of general formula I wherein $R_4$ and/or $R_5$ represents hydrogen is alkylated.

7. Pharmaceutical compositions containing as active substances compounds of general formula I combined with conventional excipients and/or carriers.

8. Pharmaceutical compositions as claimed in claim 7 for the treatment of depressive conditions of various origins.